# EUROPEAN PATENT SPECIFICATION

(11) **EP 2 575 712 B1**
(45) Date of publication and mention of the grant of the patent: **31.10.2018**
(21) Application number: 11721723.2
(22) Date of filing: 26.05.2011
(51) Int. Cl.: A61F 9/007, A61F 9/008

(54) **DEVICE FOR CATARACT SURGERY**
VORRICHTUNG ZUR KATARAKTCHIRURGIE
DISPOSITIF DE CHIRURGIE DE LA CATARACTE

(30) Priority: 27.05.2010 US 349042 P; 27.05.2010 DE 102010022298
(43) Date of publication of application: 10.04.2013
(73) Proprietor: Carl Zeiss Meditec AG, 07745 Jena (DE)
(72) Inventor: KASCHKE, Michael, 73447 Oberkochen (DE); MONZ, Ludwin, 07743 Jena (DE); DAMM, Tobias, 81545 München (DE); MÜHLHOFF, Dirk, 07751 Jena (DE); REIN, Karlheinz, 73430 Aalen (DE); STOBRAWA, Gregor, 07743 Jena (DE); DICK, Manfred, 07926 Gefell (DE); KÜHNER, Martin, 07639 Bad Klosterlausnitz (DE); HÖGELE, Artur, 73447 Oberkochen (DE); REICH, Matthias, 07749 Jena (DE); GREBNER, Dieter, 07751 Grosslöbichau (DE)
(74) Representative: Rößner, Ulrike
(86) International application number: PCT/EP2011/002608
(87) International publication number: WO 2011/147570

(56) References cited:
- EP-A1- 0 228 778
- WO-A2-2009/039302
- DE-A1- 3 724 282
- DE-A1- 19 940 712
- US-A- 4 638 801
- US-A- 4 887 592
- US-A1- 2002 049 450

## Description

### Field of application:

The invention relates to improvements in respect of performing cataract surgery, and the result thereof, by means of a laser system.

### Prior art:

To date, the following steps are typically performed on the eye during cataract surgery, which eye has been dilated by drops (i.e. the pupil has been dilated by medicaments) and is under local anesthetics:
- Making an approximately 1.5-3 mm wide cut, set manually, into the cornea using a scalpel in order to provide access to the anterior chamber of the eye for all that follows and
- making a circular cut, set manually, with a diameter of approximately 3-6 mm into the anterior capsular bag using a special scalpel; removing the capsular-bag segment in order to provide access to the lens.
- Cutting apart the lens or the lens fragments, and subsequent further subdivision thereof, combined with suctioning off the fragments using an ultrasound device/rinsing-suctioning device using different ultrasound energy levels and rinsing speeds or suction pressure levels (phacoemulsification).
- Inserting the intraocular lens into the capsular bag.
As an alternative to the previously used instruments (surgical microscope, phacoemulsification apparatus, also referred to as phaco machine), or in addition thereto, use is made of a femtosecond laser system (fs-laser) for:
- cutting into the cornea,
- cutting into the capsular bag,
- cutting apart and further subdividing of the lens,
- making optionally desired or necessary relaxing cuts into the cornea (in order to compensate for astigmatism).
The use of a cutting laser makes it possible to perform more precisely positioned cuts, which are more defined in terms of their dimensions. As a result of the additional implementation of a navigation, e.g. by means of optical coherence tomography in conjunction with the laser system, the processes of cutting and dividing can be automated, without tissue worth retaining, e.g. the posterior capsular bag, being injured. As a result, the significant risks of manual/visual cut guidance by the operator are avoided and even medical practitioners with less surgical experience achieve a lower complication rate and better refractive results.

WO09039302 describes such a laser system: a laser is guided onto the eye via a deflection mirror and an objective. An x/y/z scanner moves this laser beam in the eye and performs cuts. The z-scan can also be embodied as a displacement of the objective along the optical axis. There, OCT (optical coherence tomography) is used for navigation. An fs-laser or a ps-laser without more detailed specification is mentioned as a laser. The laser system is designed as an independent system, with the imaging required for the navigation being integrated therein. It operates independently from phaco machines and surgical microscopes. This allows the laser cuts to be able to be performed before suctioning off the lens fragments and before inserting the lens, e.g. in a different spatial area. However, this assumes a modified process organization in the hospital or the surgical practice. Alternatively, the laser system must be pushed to-and-fro in an intricate and laborious fashion in a conventionally organized operating room. An expensive fs-laser is necessary for the implementation; the aperture is restricted as a result of the large working distance, and this leads to a majority of the residual energy of the laser being radiated in the direction of the retina and constituting a safety risk.

A different laser system is described in US7621637, which is designed only for refractive corneal surgery. It is a system in which a laser is swiveled into the surgical region between the microscope lens of the surgical microscope and the eye by means of a swivel arm. A horizontal flap-cut plane is made in the cornea by a slow movement of the objective along one direction and a rapid movement of a tilting mirror, which is housed in an independent module. The advantage of this system lies in the simple integration into the corneal-surgery procedure. By way of example, the observation by the operator is only interrupted during the swiveling-in and during the flap cut.
The use of an expensive fs-laser and the long scan time by the objective scan were found to be disadvantageous, although the latter is acceptable during a corneal-flap cut because only one cut needs to be made. Furthermore, as a result of the principles thereof, the depth of focus in this system is not deep enough to be able to perform cuts in the lens, and it lacks a 3D navigation, which is necessary for lens cuts. Moreover, the overall device is too voluminous for the typical operation situation (surgical microscope, phaco machine).

Another laser system, which uses an UV laser to treat the crystalline lens of the eye by photoablation or photodisruption, is described in US 2002/049450 A1. It comprises a surgical microscope head. Both the laser system and the optical microscope head are working independently, but arrive in the eye via the same observation beam path. There is no mechanical relationship between the surgical microscope head and the laser system.

DE 37 24 282 A1 discloses a laser system for the surgery of the cornea of the human eye, which can be attached to a conventional surgical microscope in a way that the laser module is pivotable into the observation path of the surgical microscope head. There is no mechanical or optical intervention to the surgical microscope head, as such an intervention is considered to disturb the correct functioning of the surgical microscope.

### Description of the invention:

It is an object of the invention to develop a laser system for cataract surgery, which has a more compact design and can therefore be integrated more easily into the existing system surroundings, is more cost-effective but nevertheless meets the requirements of laser-lens surgery, such as large aperture (in order not to burden the retina and in order to obtain a sufficiently small focus) and high scan speed (in order to be able to perform all necessary steps, more particularly for comminuting the lens, within approximately 1 minute). This is achieved by a device for cataract surgery according to claim 1. Preferred embodiments of the invention are illustrated in the dependent claims. In a first variant (A1), this object is achieved by a laser- and navigation system as an add-on module for a surgical microscope, consisting of:
1. an fs-laser or a ps-laser;
2.
   a) a module, which can be added on, with a high-aperture objective with a large adjustable range of the focus, which module is held at the microscope such that it can swivel and can be positioned in a defined fashion between microscope objective and contact lens or eye,
   b) or, alternatively, a module, which can be added on, with a high-aperture objective, which module is held fixedly at the microscope and can be positioned in a defined fashion between microscope objective and contact lens or eye, wherein the module additionally has a lens for partial compensation of the objective effect when looking through the objective;
3. optionally a contact lens (such that the overall system, made of the objective and, optionally, the contact lens, has a NA > 0.2);
4. a deflection/diffraction unit, which couples the laser into the objective, as a component of the module. In the case 2.b), it is embodied as a dichroic mirror (transmits in the visible range, reflects in the laser-wavelength range);
5. two fast scanners with a mirroring embodiment or a fast 2-axes scanner, which deflect(s) the laser beam in the x/y direction, or a scanner and elements for beam rotation as a component of the module;
6. optionally a detection unit, which detects the laser light reflected/scattered from the eye and obtains navigation data therefrom for the online control and the pre-orientation of the cut pattern;
7. a laser feed, preferably an optical waveguide or a free-beam articulated arm, which feeds the laser beam to the module from the laser source.
This variant is particularly suitable for a large number of cuts when comminuting the lens.

In an alternative variant (A2), this object is achieved by a laser- and navigation system with add-on modules for a surgical microscope, consisting of:
1. an fs-laser or a ps-laser;
2. a module, which can be added on, with a high-aperture objective with a large adjustable range of the focus, which module is held at the microscope such that it can swivel and can be positioned in a defined fashion between microscope objective and contact lens or eye;
3. optionally a contact lens (such that the overall system, made of the objective and, optionally, the contact lens, has a NA > 0.2);
4. a unit that couples the laser into the microscope, wherein the microscope optical system couples the laser into the displaceable high-aperture-like objective;
5. a beam scanner, consisting of e.g. two fast scanners with a mirroring embodiment or a fast 2-axes scanner, which deflect(s) the laser beam in the x/y direction, or a scanner and elements for beam rotation as a component of the objective module, which can be added on, or as a separate module in the laser beam feed to the surgical microscope;
6. optionally a detection unit, which detects the laser light reflected/scattered from the eye and obtains navigation data therefrom for the online control and the pre-orientation of the cut pattern.

This variant is likewise particularly suitable for a large number of cuts when comminuting the lens, and wherein the microscope already contains coupling-in means for a laser, e.g. also via the optical interface of a co-observation optical system.

In a further alternative variant (B1), this object is achieved by a laser- and navigation system as an add-on module for a surgical microscope, consisting of:
1. an fs-laser or a ps-laser;
2. a module, which can be added on, with a high-aperture objective that can be displaced in the x/y/z-direction within the module, which module is held at the microscope such that it can swivel and can be positioned in a defined fashion between microscope objective and contact lens or eye;
3. optionally a contact lens (such that the overall system, made of the objective and, optionally, the contact lens, has a NA > 0.2);
4. a unit that couples the laser into the microscope, as a component of the module;
5. a 3-axes objective positioning unit, implemented by means of e.g. piezo-drives or stepper- or servo motors;
6. optionally a detection unit, which detects the laser light reflected from the eye and obtains navigation data therefrom for the online control and the pre-orientation of the cut pattern;
7. a laser feed, preferably an optical waveguide or a free-beam articulated arm, which feeds the laser beam to the module from the source.
This variant is particularly suitable for a relatively small number of cuts, e.g. if only a cross cut of the lens is intended to be cut, or else if only tough, dense cataract regions are intended to be cut.

In a further variant (B2), this object is achieved by a laser- and navigation system with add-on modules for a surgical microscope, consisting of:
1. an fs-laser or a ps-laser;
2. a module, which can be added on, with a high-aperture objective that can be displaced in the x/y/z-direction within the module, which module is held at the microscope such that it can swivel and can be positioned in a defined fashion between microscope objective and contact lens or eye;
3. optionally a contact lens (such that the overall system, made of the objective and, optionally, the contact lens, has a NA > 0.2);
4. a unit that couples the laser into the microscope, wherein the microscope optical system couples the laser into the displaceable high-aperture objective;
5. a 3-axes/3D objective positioning unit, implemented by means of e.g. piezo-drives or stepper- or servo motors. As an alternative to the x/y or x-scan, the objective can also be tilted by motor in the x/y axes or in the x-axis;
6. optionally a detection unit as an add-on for the microscope or the module, which can be added on, which detection unit detects the laser light reflected from the eye and obtains navigation data therefrom for the online control and the pre-orientation of the cut pattern;
7. a laser feed, preferably an optical waveguide or a free-beam articulated arm, which feeds the laser beam to the microscope from the source.

This variant is particularly suitable for a relatively small number of cuts, e.g. if only a cross cut of the lens is intended to be cut, or else if only tough, dense cataract regions are intended to be cut and when the surgical microscope already contains coupling-in means for a laser, e.g. also via the optical interface of a co-observation optical system.

Here, the fs-laser preferably has a pulse duration of between 100 fs and 1000 fs, with a pulse energy of between 0.10 µJ and 10.00 µJ and repetition frequencies of between 50 kHz and 500 kHz. What this energy and these pulse durations achieve is that the laser can produce a vapor/plasma bubble in a tissue volume with a diameter of approximately 5 micrometers, with only small effects being induced outside of this volume. The high repetition frequency, in conjunction with the fast scanners, affords the possibility of, within one minute, being able to produce at least 8 vertical/or radial and 2 horizontal cuts in the lens, which has a thickness of between 3-6 mm.
The ps-laser preferably has a pulse duration of between 1 ps and 20 ps, with a pulse energy of between 1 µJ and 200 µJ and repetition frequencies of between 25 kHz and 150 kHz. What this energy and these pulse durations achieve is that the laser produces a vapor/plasma bubble in a tissue volume with a diameter of approximately 10-15 micrometers. However, unlike the fs-laser, the ps-laser affects a larger volume on account of the thermal effects and pressure effects. However, this is largely uncritical, seeing as the lens is in any case removed entirely during the cataract operation and the cuts to the cornea and in the capsular bag need not satisfy the precision (e.g. in respect of coarseness, dimensional accuracy) required for optical imaging. However, the pulse duration and energy should not lie substantially above the specified values because otherwise there is a significant increase in the risk of injury to the corneal endothelial cells by pressure peaks or to the anterior capsular bag segments remaining in the eye after the intervention.

What holds true for both lasers is that in the case of relatively small lenses or if the cuts are only performed in the dense regions of the lens or if the cuts only serve to replace the initial ultrasound cross cut, fewer cuts than the aforementioned approximately 8 are also expedient, and so the repetition frequency or the overall time for the cuts may be reduced.
However, in the case of a dense or tough cataract or cataract regions - identifiable e.g. from the stray-light data from the detector unit - the number of cuts overall may also be increased, or else there may be a local increase in the cut density only in the dense or tough cataract regions. In particular, the distance between 2 cut surfaces can be reduced to the typical geometry/dimension of the suction-head inlet, or to less than that. As a result, the fragments of the lens need not be comminuted much more by a subsequent phaco step. This can significantly reduce the ultrasound energy or, ideally, this may allow the use of ultrasound to be dispensed with entirely.

In a method for laser-assisted eye surgery not according to the present invention, the laser-beam source is flexibly connected to a scanning and focusing module, which, mechanically balanced in direct contact with the eye, is used for intraocular navigation and therapy.
The invention will be explained in more detail in the following text on the basis of the drawings, in which:
- figure 1: shows a schematic illustration of the invention according to variant A1,
- figure 2: shows a schematic illustration of the invention according to variant A1 in a 2^{nd} embodiment,
- figure 3: shows a schematic illustration of the invention according to variant A2,
- figure 4: shows a schematic illustration of the invention according to variant A2 in a 2^{nd} embodiment,
- figure 5: shows a schematic illustration of the invention according to variant B1, and
- figure 6: shows a schematic illustration of the invention according to variant B2.

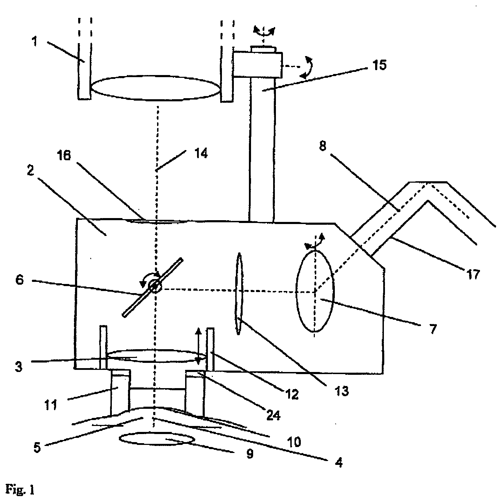

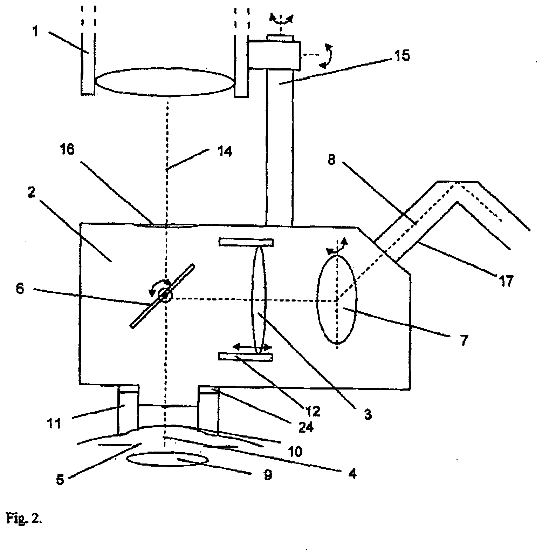

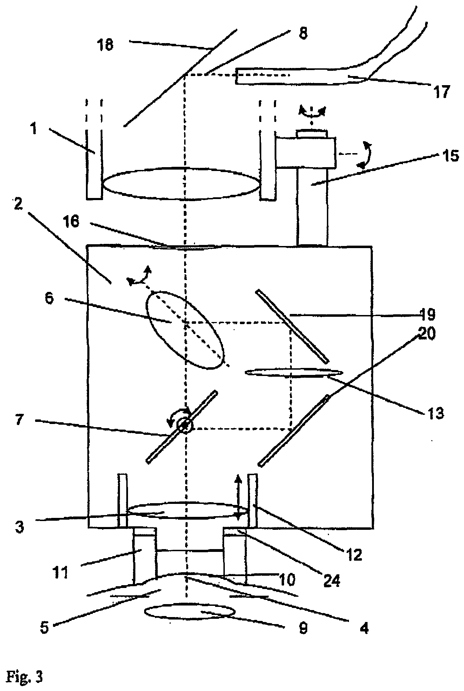

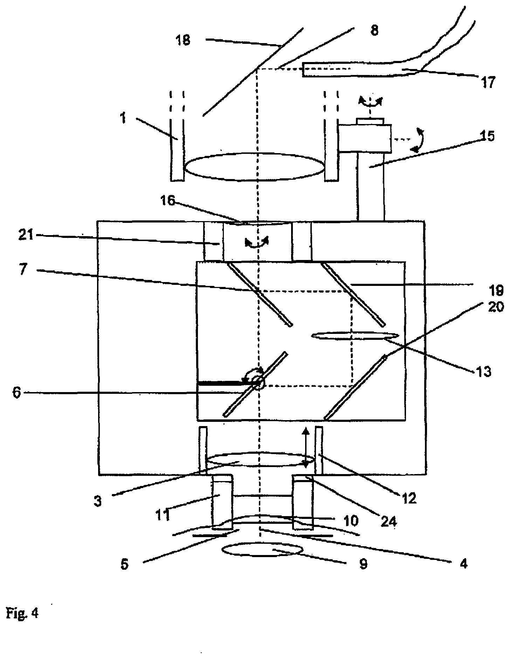

Figure 1 illustrates the invention according to variant A1. It contains the module 2, which can be added on to a surgical or stereo microscope 1, an objective 3 with great displacement of the focus along the optical axis 4 of the eye 5, and a deflection unit 6, 7 for coupling in the laser beam 8, wherein the deflection unit 6, 7 is embodied as a scanning unit for deflecting the laser in the x/y-direction.
In one focusing position, the objective 3 is able to focus onto the rear side of the lens 9 of the eye and, in another focusing position, said objective is able to focus onto the front side of the lens 9 of the eye; advantageously it is additionally also able to focus onto the front side of the cornea 10. The focus drive 12 serves to shift the position of the focus. At the same time, the objective 3 must be able to cover a scanning field with the diameter of a pupil dilated by drops or from the center of the pupil to the sclera. It must likewise have an aperture large enough to ensure that the light is sufficiently defocused on the retina so that no injury threshold of the retina is exceeded by the light cone during a treatment duration of approximately 1 minute. That is to say the through-focusing region is greater than 10-12 mm, at least greater than 1 mm, in a field with a diameter of greater than 4 mm and an aperture of greater than approximately 0.20. In order to achieve this in the case of a small overall size and low weight, aspherical or free-form lens surfaces and/or diffractive elements and/or a contact lens 12 (with planar contact surface, or a contact surface matched to the corneal curvature) and/or adaptive mirror surfaces may be used. The contact lens 11 is affixed on the eye by means of negative pressure.
By way of example, 2 fast galvo-scanners are options for the scanners 6, 7. However, a scanner that scans along the meridian and is itself mechanically rotated, or the beam of which is rotated, by e.g. a prism, (so that there is a meridian-rotation) is also an alternative option. As a further alternative option, use can also be made of a MEMS scanner that can move along 2 axes. In the embodiment illustrated in figure 1, the objective 3 of the module 2 is in the imaging beam path of both the laser (not illustrated here) and the microscope 1. An advantage of this is that the aperture of the objective 3 can be selected to be very large; a relay lens 13 serves for matching the laser beam 8 to the observation beam path 14 of the microscope 1.
However, the objective 3 may also be installed in the beam path of the laser, upstream of where said beam path merges into the observation beam path 14 of the microscope 1, e.g. between the two scan mirrors 6, 7, as illustrated in figure 2. This affords the possibility of better independent regulation of the foci of laser and observation beam path 14. According to the present invention, a fastener 15 connects the module 2 to the microscope 1 e.g. such that it can swivel. The module furthermore has an entry window 16 for the observation beam path 14, which entry window may also be embodied as a matching lens. The laser beam 8 is coupled into the module 2 via a feed 17, which may be embodied as a fiber or else as a free-beam apparatus.
In variant A2, the laser is firstly coupled into the microscope, and the latter transmits the laser into the objective 3. This is illustrated in figure 3. Hence the microscope 1 itself has the deflection or beam splitter unit 18. Scan elements 6, 7 for laser-beam scanning can be positioned upstream of where the laser is coupled into the microscope or, as illustrated, between the microscope 1 and the objective 3. The scan apparatus moreover has 2 additional fixed reflectors 19, 20 and a relay lens 13. Otherwise, identical elements in figure 3 have identical reference signs as in figures 1 and 2; reference is made to the description relating to these. Figure 4 shows a further embodiment of the variant A2. Here, use is made of only one movable deflection element 6; deflection element 7 is fixed. The required second movement direction of the scanned laser beam 8 is implemented by rotation of the entire module 2 about the optical axis 4 by means of the circular guide 21. The position of the deflection elements 6, 7 may also be interchanged with the position of the reflectors 19, 20 in both figure 3 and figure 4.

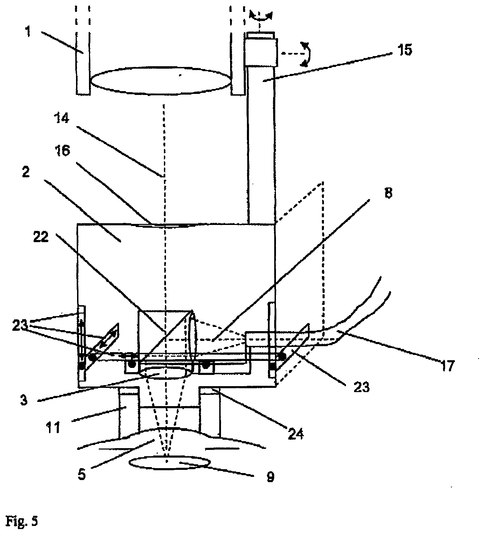

In variants B1 and B2, the module 2, which can be added on to a surgical or stereo microscope 1, contains an objective 3 with mechanical displacement of the focus both along the optical axis of the eye and laterally in the x/y-direction. Here the same conditions for the necessary adjustment tracks of the focus of the objective 3 hold true as in the variants A1 and A2. Figure 5 illustrates an embodiment of the module 2 according to variant B1. Here, the laser beam 8 is coupled into the module 2 via a deflection unit 22 (as in figures 1 and 2). The three-dimensional movement of the focus of the laser beam 8 is implemented by an x/y/z-movement of the objective 3, e.g. along guides 23 by means of piezo-elements or stepper- or servo motors, with or without position feedback.

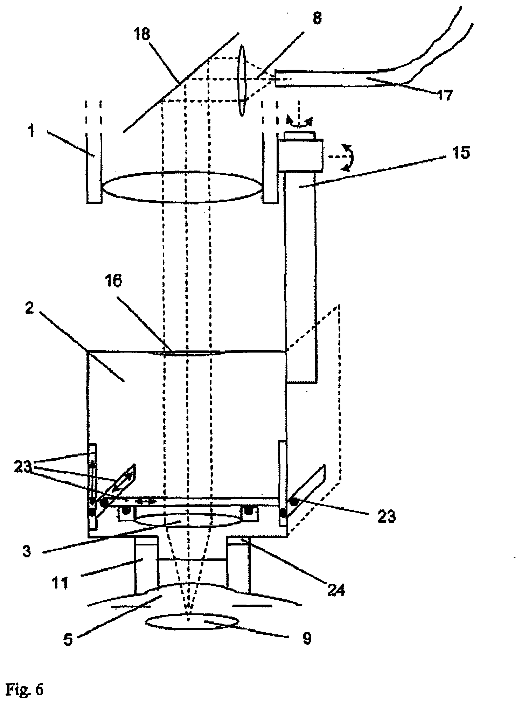

Figure 6 shows an embodiment according to variant B2. Here, the laser beam 8 is firstly coupled into the microscope 1, and from there it is coupled into the objective 3 of the module 2. As in figure 5, the focus adjustment is implemented by 3-dimensional movement of the objective 3 by means of the guides 23. Unlike variant B1, the beam cone in the object, e.g. the lens of the eye, impinges obliquely on the object for x/y focus positions away from the optical axis of the eye and is therefore subject to less interference by the iris.

A confocal detector (not illustrated here) or a planar detector serves as a detection unit. The confocal signal serves for determining the boundaries, as is described in DE 103 23 422, the entire content of which being referred to. Together with the non-confocal component, this allows a scattering intensity to be determined, and this scattering intensity can be used to control the laser in terms of one or even more of the following parameters: pulse energy, pulse duration, repetition frequency and/or scan speed. The reflected light may optionally be decoupled via a combination of wave plate and polarization splitter. An OCT unit may also be considered as detection unit.

So that the overall module 2 is made manageable from a mechanical standpoint for an operator, the structural elements in the module 2 are arranged and distributed such that the center of gravity of the module 2 is situated below the objective of the surgical microscope head 1, but it is at least situated along the nadir from the center of gravity of said microscope head. Furthermore, a device 24 for generating a minimum contact pressure of the module 2 on the contact lens 11 is integrated into the module 2. This can be implemented by a pressure transducer, e.g. by a spring or an electromechanical pressure actuator, which, from the module, presses against the contact lens or the eye with a defined force, the latter optionally being fed-back via a sensor. This device can also be able to move (pressure-distance transducer) the contact lens 11 in the direction of the eye over the small distances (1 mm).

In order to aid integration and simplify the operation, provision is made - integrated into the module or provided externally - for a controller/control unit that supports the following operational procedure:
1. The operator optionally (a) places the contact lens on the eye, which has been dilated by drops and is anesthetized, or, alternatively, (b) the contact lens is placed onto the add-on module.
2. The operator moves the microscope head over the contact lens or eye. The lateral position and the position relating to the distance (size of the centering) from the contact lens or the eye can be set by said operator by means of a centering, which is reflected in or identified by means of a monitor image. The module is swiveled in manually or automatically once a suitable distance is established; in order to establish the contact between the module and contact lens in case (a), the microscope head is displaced to the eye and/or the pressure-distance transducer is lengthened, until there is the necessary contact with the eye and the necessary contact pressure thereon. Thus suction pressure is applied during this or thereafter. In order to be able to establish the contact between the module/contact lens and eye in case (b), the microscope or the pressure-distance transducer can likewise be utilized in a fashion that is analogous to case (a).
3. The laser system scans the eye area in 3D at low energy levels. The detection unit determines landmarks and the envisaged cut pattern is oriented on the real 3D structure. The cut spacing in accordance with the cataract density is optionally obtained from the detector data and modified such that the remaining fragments can be destroyed at low ultrasound energy and can be suctioned in.
4. The laser cuts apart the lens from posterior to anterior on the basis of the oriented cut pattern and under online navigation/laser-parameter monitoring.
5. The laser cuts apart the capsular bag.
6. The laser makes the corneal cut, optionally after a preceding repeated 3D orientation.
7. Contact pressure and suction pressure are removed. The microscope and/or the pressure-distance transducer are pulled away from the eye. The module is swiveled away manually or automatically. The contact lens is removed manually.
8. Optionally, information relating to the position of the cuts, particularly in the cornea and in the capsular bag, is displayed on the monitor image or reflected into the OPMI.
9. The operator manually continues the cataract surgery.

The invention is not restricted to the illustrated exemplary embodiments; developments by a person skilled in the art do not depart from the scope of protection.

## Claims

1. A device for cataract surgery, comprising a surgical microscope head (1) or stereo microscope head and a laser source,
- in which a module (2), consisting of a laser-coupling/deflecting unit, and/or a laser-scan unit (6,7), and a focusing unit (3), can be mechanically coupled to the surgical microscope head (1) or stereo microscope head by a fastener (15), and can be moved by the surgical microscope head (1) or stereo microscope head,
- in which at least one of these units can selectively be introduced in an observation beam path (14) between the surgical microscope head (1) or stereo microscope head and an eye (5),
- and in which the focusing unit (3) can scan a depth-of-focus range of greater than 1 mm.

2. The device as claimed in claim 1, in which the focusing unit (3) contains at least one aspherical surface or at least one diffractive element or an adaptive element.

3. The device as claimed in one of the preceding claims, in which the center of gravity of the module (2) attached to the surgical microscope head (1) or stereo microscope head
- is situated below the center of gravity of the surgical microscope head (1) or stereo microscope head, or
- coincides with the center of gravity of the microscope (1) head or stereo microscope head, or
- is situated along the nadir from the center of gravity of the surgical microscope head or stereo microscope head.

4. The device as claimed in one of the preceding claims, in which two reflecting scanners, or a scanner and a rotation element, or a 2-axes scanner, or the objective are integrated in the module for deflecting a laser beam or for displacing the laser beam focus laterally in 2 dimensions.

5. The device as claimed in claim 4, in which one of the scanners is simultaneously embodied as deflection unit.

6. The device as claimed in one of the preceding claims, in which the surgical microscope head (1) or stereo microscope head itself comprises the laser-coupling/deflecting unit and the surgical microscope head (1) or stereo microscope head couples the laser into the focusing unit.

7. The device as claimed in one of the preceding claims, in which an fs-laser, with a pulse duration of 100-1000 fs, or a ps-laser, with pulse durations of 1-20 ps, is utilized as the laser source.

8. The device as claimed in one of the preceding claims, in which a detection unit detects the light, which has been reflected in a confocal and/or planar fashion, and utilizes said light for the pre-orientation of a desired cut pattern, a navigation or a laser-parameter control.

9. The device as claimed in claim 8, in which the detection unit is integrated into the module.

10. The device as claimed in claim 8, in which the reflected light and the current data obtained therefrom are compared to data from the biometry obtained preoperatively.

11. The device as claimed in one of the preceding claims, in which a contact lens is used in addition to the objective, wherein the contact lens is configured to couple the module which is attached to the surgical microscope head or stereo microscope head to the eye.

12. The device as claimed in one of the preceding claims, in which adjustment means for positioning the surgical microscope head or stereo microscope head and the module over the contact lens or the eye are provided in or on the surgical microscope head or stereo microscope head and the module.

13. The device as claimed in one of the preceding claims, in which contact-pressure and/or suction devices or contact-pressure and/or suction pressure transmission lines are fitted onto/into the module.

## Patentansprüche

1. Vorrichtung zur Kataraktchirurgie, umfassend einen Operationsmikroskop-Kopf (1) oder Stereomikroskop-Kopf und eine Laserquelle,
- wobei ein Modul (2), das aus einer Laserkopplungs-/-umlenkeinheit, und/oder einer Laser-Scan-Einheit (6, 7), und einer Fokussiereinheit (3) besteht, durch ein Befestigungsmittel (15) mechanisch mit dem Operationsmikroskop-Kopf (1) oder Stereomikroskop-Kopf verbunden werden kann und vom Operationsmikroskop-Kopf (1) oder Stereomikroskop-Kopf bewegt werden kann,
- wobei mindestens eine dieser Einheiten selektiv in einen Beobachtungsstrahlengang (14) zwischen dem Operationsmikroskop-Kopf (1) oder Stereomikroskop-Kopf und einem Auge (5) eingeführt werden kann,
- und wobei die Fokussiereinheit (3) einen Fokustiefenbereich von mehr als 1 mm scannen kann.

2. Vorrichtung nach Anspruch 1, wobei die Fokussiereinheit (3) mindestens eine asphärische Oberfläche oder mindestens ein diffraktives Element oder ein adaptives Element enthält.

3. Vorrichtung nach einem der vorhergehenden Ansprüche, wobei der Schwerpunkt des Moduls (2), das am Operationsmikroskop-Kopf (1) oder Stereomikroskop-Kopf befestigt ist,
- unter dem Schwerpunkt des Operationsmikroskop-Kopfs (1) oder Stereomikroskop-Kopfs angeordnet ist, oder
- mit dem Schwerpunkt des Mikroskop-Kopfs (1) oder Stereomikroskop-Kopfs zusammenfällt, oder
- entlang des Lots vom Schwerpunkt des Operationsmikroskop-Kopfs oder Stereomikroskop-Kopfs angeordnet ist.

4. Vorrichtung nach einem der vorhergehenden Ansprüche, wobei zwei reflektierende Scanner, oder ein Scanner und ein Rotationselement, oder ein 2-Achsen-Scanner, oder das Objektiv im Modul integriert sind, um einen Laserstrahl abzulenken oder um den Laserstrahlfokus lateral in 2 Dimensionen zu verschieben.

5. Vorrichtung nach Anspruch 4, wobei einer der Scanner gleichzeitig als Umlenkeinheit ausgelegt ist.

6. Vorrichtung nach einem der vorhergehenden Ansprüche, wobei der Operationsmikroskop-Kopf (1) oder Stereomikroskop-Kopf selbst die Laserkopplungs-/- umlenkeinheit umfasst, und der Operationsmikroskop-Kopf (1) oder Stereomikroskop-Kopf den Laser in die Fokussiereinheit einkoppelt.

7. Vorrichtung nach einem der vorhergehenden Ansprüche, wobei ein fs-Laser mit einer Pulsdauer von 100-1000 fs oder ein ps-Laser mit Pulsdauern von 1-20 ps als Laserquelle verwendet wird.

8. Vorrichtung nach einem der vorhergehenden Ansprüche, wobei eine Detektionseinheit das Licht erkennt, das auf konfokale und/oder planare Weise reflektiert wurde, und dieses Licht für die Vororientierung eines gewünschten Schnittmusters, einer Navigation oder einer Laserparametersteuerung verwendet.

9. Vorrichtung nach Anspruch 8, wobei die Detektionseinheit im Modul integriert ist.

10. Vorrichtung nach Anspruch 8, wobei das reflektierte Licht und die daraus erhaltenen aktuellen Daten mit präoperativ erhaltenen Daten aus der Biometrie verglichen werden.

11. Vorrichtung nach einem der vorhergehenden Ansprüche, wobei ein Kontaktglas zusätzlich zum Objektiv verwendet wird, wobei das Kontaktglas dazu ausgelegt ist, das Modul, das am Operationsmikroskop-Kopf oder Stereomikroskop-Kopf befestigt ist, am Auge anzukoppeln.

12. Vorrichtung nach einem der vorhergehenden Ansprüche, wobei Justiermittel zur Positionierung des Operationsmikroskop-Kopfs oder Stereomikroskop-Kopfs und des Moduls über dem Kontaktglas oder dem Auge in oder auf dem Operationsmikroskop-Kopf oder Stereomikroskop-Kopf und dem Modul vorgesehen sind.

13. Vorrichtung nach einem der vorhergehenden Ansprüche, wobei Anpressdruck- und/oder Ansaugvorrichtungen oder Anpressdruck- und/oder Ansaugdruck-Übertragungsleitungen auf/in dem Modul angebracht sind.

## Revendications

1. Dispositif pour la chirurgie de la cataracte, comprenant une tête de microscope chirurgical (1) ou une tête de microscope stéréoscopique et une source laser,
- dans lequel un module (2), se composant d'une unité de couplage laser/de déviation et/ou d'une unité de balayage laser (6, 7) et d'une unité de focalisation (3) peut être couplé mécaniquement à la tête de microscope chirurgical (1) ou à la tête de microscope stéréoscopique par un dispositif de fixation (15) et peut être déplacé par la tête de microscope chirurgical (1) ou par la tête de microscope stéréoscopique,
- dans lequel au moins l'une de ces unités peut être introduite de manière sélective dans un trajet de faisceau d'observation (14) entre la tête de microscope chirurgical (1) ou la tête de microscope stéréoscopique et un oeil (5),
- et dans lequel l'unité de focalisation (3) peut balayer une plage de profondeur focale supérieure à 1 mm.

2. Dispositif selon la revendication 1, dans lequel l'unité de focalisation (3) contient au moins une surface asphérique ou au moins un élément de diffraction ou un élément adaptatif.

3. Dispositif selon l'une des revendications précédentes, dans lequel le centre de gravité du module (2) fixé à la tête de microscope chirurgical (1) ou à la tête de microscope stéréoscopique
- est situé en dessous du centre de gravité de la tête de microscope chirurgical (1) ou de la tête de microscope stéréoscopique ou
- coïncide avec le centre de gravité de la tête de microscope (1) ou de la tête de microscope stéréoscopique ou
- est situé le long du nadir du centre de gravité de la tête de microscope chirurgical ou de la tête de microscope stéréoscopique.

4. Dispositif selon l'une des revendications précédentes, dans lequel deux dispositifs de balayage réfléchissants, ou un dispositif de balayage et un élément de rotation, ou un dispositif de balayage à 2 axes ou l'objectif sont intégrés dans le module pour dévier un faisceau laser ou pour déplacer le foyer du faisceau laser latéralement dans 2 dimensions.

5. Dispositif selon la revendication 4, dans lequel l'un des dispositifs de balayage est intégré en même temps sous la forme d'une unité de déviation.

6. Dispositif selon l'une des revendications précédentes, dans lequel la tête de microscope chirurgical (1) ou la tête de microscope stéréoscopique comprend elle-même l'unité de couplage laser/de déviation et la tête de microscope chirurgical (1) ou la tête de microscope stéréoscopique couple le laser dans l'unité de focalisation.

7. Dispositif selon l'une des revendications précédentes, dans lequel un laser fs, ayant une durée de pulsation comprise entre 100 et 1 000 fs, ou un laser ps, ayant des durées de pulsation comprises entre 1 et 20 ps, est utilisé comme source laser.

8. Dispositif selon l'une des revendications précédentes, dans lequel une unité de détection détecte la lumière, qui a été réfléchie de manière confocale et/ou plane, et utilise ladite lumière pour la pré-orientation d'un motif découpé souhaité, une navigation ou une commande de paramètre laser.

9. Dispositif selon la revendication 8, dans lequel l'unité de détection est intégrée dans le module.

10. Dispositif selon la revendication 8, dans lequel la lumière réfléchie et les données actuelles obtenues de cette dernière sont comparées à des données provenant de la biométrie obtenue de manière préopératoire.

11. Dispositif selon l'une des revendications précédentes, dans lequel un verre de contact est utilisé en plus de l'objectif, dans lequel le verre de contact est configuré pour coupler le module qui est fixé à la tête de microscope chirurgical ou à la tête de microscope stéréoscopique à l'oeil.

12. Dispositif selon l'une des revendications précédentes, dans lequel des moyens pour positionner la tête de microscope chirurgical ou la tête de microscope stéréoscopique et le module sur le verre de contact ou l'oeil sont disposés dans ou sur la tête de microscope chirurgical ou la tête de microscope stéréoscopique et le module.

13. Dispositif selon l'une des revendications précédentes, dans lequel des dispositifs de pression de contact et/ou d'aspiration ou des lignes de transmission de pression de contact et/ou de pression d'aspiration sont incorporés sur/dans le module.
